Europäisches Patentamt

(19) European Patent Office

Office européen des brevets

(11) Publication number: **0 307 373 B1**

## EUROPEAN PATENT SPECIFICATION

(45) Date of publication of patent specification: **18.11.93**  (51) Int. Cl.⁵: **C07K 3/26**, A61K 35/30, A61K 37/36, B01D 61/14

(21) Application number: **88830362.5**

(22) Date of filing: **09.09.88**

The file contains technical information submitted after the application was filed and not included in this specification

(54) **Purification process.**

(30) Priority: **11.09.87 US 96561**

(43) Date of publication of application:
**15.03.89 Bulletin 89/11**

(45) Publication of the grant of the patent:
**18.11.93 Bulletin 93/46**

(84) Designated Contracting States:
**AT BE CH DE ES FR GB GR IT LI LU NL SE**

(56) References cited:
**EP-A- 0 209 331**
**EP-A- 0 219 295**
**GB-A- 2 134 528**

**J. Tateishi et al, Lancet, 1985, 1299-1300**

**D.M. Taylor, Lancet, 1986, 559**

**Taylor, Lancet, 1985, 1430-1431**

(73) Proprietor: **ARES TRADING S.A.**
**Chateau de Vaumarcus**
**CH-2028 Vaumarcus(CH)**

(72) Inventor: **Eshkol, Alisa**
**4, Rue du Midi**
**CH-1196 Gland CH(CH)**
Inventor: **Maillard, François**
**Le Verger**
**CH-1111 Lussy s/Morges(CH)**
Inventor: **Stiles, Gerald E.**
**66 Prior Farm Road**
**Duxbury, MA 02332(US)**

(74) Representative: **Froud, Clive et al**
**ELKINGTON AND FIFE,**
**Prospect House,**
**8 Pembroke Road**
**Sevenoaks, Kent TN 13 1XR (GB)**

**Description**

Technical Field of Invention

This invention relates to a process for purifying a biological material or a product derived therefrom. More particularly, it relates to a process for the preparation of a biological extract substantially free from impurities containing viruses or virus like particles.

Background

The purification of biological fluids using ultrafiltration has been fraugth with problems due to the similarity of molecular weights of the various components of such fluids. While concentration (e.g., the isolation of fluids in which the ratio of larger molecules to smaller ones is increased) is known, the use of purely physical techniques to remove or completely separate larger molecules from smaller ones via ultrafiltration has not been documented.

The concentration of viruses by means of ultrafiltration techniques has been described in:

J.D. Gangemi et al. "Arenovirus Concentration by Molecular Filtration", Applied and Environmental Microbiology, vol. 34, No. 3 (Sept., 1977) pp. 330.2;

G.P. Shibley et al. "New Method for Large-Scale Growth and Concentration of the Epstein-Barr Viruses", Applied and Environmental Microbiology, vol. 40, No. 6 (Dec., 1980) pp. 1044-1048; and

L.E. Mathes et al. "Purification of Infectious Feline Leukemia Viruses from Larger Volumes of Tissue Culture Fluids", Journal of Clinical Microbiology, vol. 5, No. 3 (Mar., 1977) pp. 372-4. EP-A-0 219 295; TATEISHI et al, Lancet, 1985, 1299; TAYLOR, Lancet, 1985, 559; TAYLOR, Lancet, 1985, 1430 disclose a membrane filtration process to remove Creutzfeldt-Jakob disease pathogen in growth hormone preparation.

These publications and all others recited herein are hereby incorporated by reference.

The Invention

It has been discovered that viruses and other contaminants of similar size and/or molecular weight can be removed or isolated from biological fluids, e.g., extracts from urine, pituitary glands, other organs, and the like, using a physical separation technique which employs a specific type of filtering membrane.

More particularly, the present invention provides a process for substantially reducing the viral infectivity of or removing viral contaminants from a biological product without significant loss thereof characterised in that it comprises the tangential flow filtration of a fluid containing the biological product through an ultrafiltration membrane having a 100,000 dalton cut-off and the recovery of the biological product from the filtrate.

Preferably the filtering membrane having 100,000 dalton cut-off has the following specifications:

| Retention: | Blue Dextran | ≥ 99% |
|---|---|---|
| | IgG | ≥ 95% |
| Passage: | Albumin | ≥ 60% |

More specifically the filtering membrane is a polysulfone membrane.

Generally, the filtered biological product is contacted with urea.

In a preferred embodiment, a 100,000 dalton cut-off ultrafiltration "PTHK" membrane, from Millipore Corp., Bedford MAS, is used to remove retrovirus and slow virus (i.e., AIDS, CJD and the like) contaminants from pituitary extracts which contain human growth hormone (hGH). The process permits product recoveries of about 90% to about 99%.

Advantages

The process of the invention has several advantages over other methods of purifying biological fluids. It is much simpler than chemical and/or thermal operations. Thus, the cost and energy expenditures normally associated with conventional chemical and/or heat treatments are avoided.

Furthermore, fluids treated in accordance with the invention are ready for use either alone or in combination with other conventional agents, e.g., phosphate-buffered saline, for administration to humans or other animals, preferably mammals.

Other advantages and aspects of the invention will become apparent from the following description of the invention.

Description of the Invention

The invention deals with a process for purifying a product of biological origin, i.e., a material derived from body fluids/organs, which process comprises the step of filtering the impure product in fluid form, e.g. solution, using a filtration membrane which has a 100,000 dalton cut-off.

Stated differently, the invention is concerned with the removal of viruses from biological extracts using an ultrafiltration membrane having certain characteristics.

Unless stated otherwise, all percentages given herein are weight percentages and are based on total composition weight.

The Filtration System

The filtration system used in the process of the invention involves an ultrafiltration membrane. Tangential flow is also involved, e.g. in embodiments employing cassette filtration systems.

Suitable devices for carrying out the invention are commercially available from Millipore Corporation of Bedford, Massachusetts, U.S.A..

Tangential flow filtration is a known technique for the separation of larger (retentate) molecules from smaller (filtrate) molecules. In biological processing schemes, the process has been used to either collect the filtrate, which passes through the membrane, or concentrate the retentate, which does not.

While it is known to produce concentrates of virus molecules and/or other "impurities" from biological fluids, it is not known to effectively remove them therefrom, so that a highly purified filtrate liquid is obtained, i.e., with filtration efficiency of 90% to 95% or greater.

Filtration efficiency indicates the ratio of the quantity of filtrate to the initial quantity of fluid or extract. Thus,

$$\% \text{ filtration efficiency} = \frac{\text{wt. of filtrate}}{\text{wt. of impure fluid}} \times 100.$$

In removal technique--as opposed to concentration techniques--filtration efficiency is relatively low, about 40 to about 50%. This is because a concentrate of retained material (i.e., retentate) contains a significant quantity of filtrate. Thus, such a concentrate may contain 60 to 90 wt. % retentate and 10 to 40 wt. % filtrate.

In contrast thereto, the purification or removal techniques of the invention result in very small quantities of filtrate remaining with the retentate, i.e., about 1 ppm. or less of biological fluid is lost when the viral or other contaminants are trapped in or on the filtration membrane. In a preferred system, several washings, e.g., about eight or more, are made of the retentate. These contaminants, i.e., the retentate after the washing operation, are then discarded.

The filtration membrane employed in a preferred embodiment of the present invention is an ultrafiltration membrane having 100,000 dalton cut-off which is sold commercially in disc and cassette systems for use in treating biological and pharmaceutical materials. The membrane itself is designated "PTHK" by Millipore Corp. and is a polysulfone membrane cast on a polypropylene sealent using 20% glycerine. It is preserved with formaldehyde.

One preferred membrane is the Millipore PTHK ultrafiltration membrane. Its specifications are:

| Retention: | Blue Dextran | ≧ 99% |
|---|---|---|
| | IgG | ≧ 95% |
| Passage : | Albumin | ≧ 60% |

Retention markers are consistent at 1-100 psi average transmembrane pressure, with passage manner performance dependent upon both pressure and solute.

Integrity

Less than 2.00 ml/minute air diffusion per ft$^2$ at 5 psi air pressure tested on fully wetted membrane.

Chemical Compatibility

The membrane is compatible with aqueous solutions and with alcohol to 20%. Its useful pH range is 1-14. It is regenerable with NaOH up to 1.0 M. Its temperature limit is 50 degrees centigrade and it can be depyrogenated with NaOH or NaOCl.

This preferred membrane is discussed in several publications of the Millipore Corporation: AD 841, OM 131, OM 141, and UF 009.

The membranes can be configured in systems with either discs or cassettes. The membrane functions in the invention in either environment. However, it is preferred that the cassette systems be employed. The manufacturer, Millipore, designates the cassette systems as "Pellicon" cassette systems (for larger volumes) or "Minitan" systems (for smaller volumes). Disc membranes are used in a stirred cell system, under pressure.

Other useful filtration systems include SM16527 of Sartorius and Filtron and the like.

Filtration Parameters

The biological fluids to be treated are pumped through the filtration system at the rate of about 300 to about 500ml/minute or more.

The membrane used can generally be described as having a surface of 0.46 m$^2$/each cassette. Of course more than one cassette can be employed; in this case the flow rate through the system has to be increased accordingly. For the Minitan system, surface area values of about 120 to about 600 cm$^2$ are common. For the Pellicon system, values of about 465 to about 46,500 cm$^2$ are the case. These ranges depend upon the number of cassettes used.

The pressure employed will vary from about 0.2 to about 1.5 bar, with 0.3 to 1.0 bar preferred but higher pressure can also be employed.

Temperature is not critical. However, it is generally preferred that the filtration process take place at a temperature of about 2 degrees to about 10 degrees centigrade or celsius.

The ratio between retentate and filtrate flow rates is a function of the backpressure used. It can vary from about 30 to about 60%, with a ratio of about 50% preferred.

Biological Fluids

The biological fluids to be treated in accordance with the invention, i.e., from which viral and/or other contaminants are to be removed, include a wide variety of liquid and semi-liquid materials which are derived from humans or animals, i.e., tissues or fluids derived from body parts. The preferred donor or source organisms for such tissues are humans and other mammals. However, tissue and other fluids from non-mammalian sources can be treated.

It is preferred that the extracts or other biological fluids to be treated be obtained from the physical treatment of tissues and body fluids which are produced via the normal metabolism of the host organism. Thus, blood, urine, and the like, can also be treated.

In addition, preparations derived using recombinant techniques, e.g., containing hormones such as rec-hGH, can also be purified using this method. Thus cell cultures, e.g., those containing genetically-engineered mammalian and/or viral cells, can be treated in accordance with the invention. Preparations containing hormones, and the like, e.g., FSH, LH, HCG, hGH, EGF interferons, etc. are preferred materials to be treated in accordance with the invention.

The type of tissue from which the fluid or extract is derived is not critical. However, it is generally preferred that the tissue be derived from body members, e.g., mammalian organs or glands via conventional extractions techniques. Thus extracts from the brain, liver, heart etc. of any mammal are preferred. Extracts from the brain--and, in particular, the pituitary glands--of human beings, hamsters, mice, rats and the like are preferred. Mixtures of extracts can be used.

The extraction technique used to remove the fluid to be treated from the tissue, other cellular material or body fluid of the invention is not critical. However, it is generally preferred that the fluid be obtained via steps such as solvent or salt precipitation, ion-exchange chromatography, size exclusion chromatography and the like. The fluid to be treated must be free of particles, e,.g., having been clarified on, for example, a

4

0.45 micron filter or other suitable filtration device.

Solutes and/or pH stabilizers such as urea, NaOH NH$_4$HCO$_3$ and phosphate buffer are usually present in the biological fluids to be treated. Mixtures are operable. A wide variety of solutes can be used. However, those solvents which after the effectiveness of the membranes should be avoided.

Suitable quantities of urea and/or decontaminating agents can also be used to deactivate any contaminant(s) remaining in the fluid after treatment. The effect of urea on the reduction of infectivity of Scrapie and CJD agents has been investigated in the past. Most investigators (Hunter et al., 1986, Kimberlin and Walker, 1985, March et al., 1984, Prusiner 1982, and Mould, unpublished data 1969), in spite of the fact that their experimental procedures diferred, found a reduction of 2-3 logs of infectivity. Applicants' results, as well as those cited above, are divergent from the observations of Brown et al. (1986), who did not observe a significant effect from the addition of urea to 20% brain homogenates of Scrapie infected hamsters or CJD infected guinea pigs.

In the past, the use of NaOH for the inactivation of infectivity has been recommended. However, NaOH causes partial inactivation and modification of the hGH molecule, whereas exposure to urea at concentrations of about 2 to about 8 M, preferably using solutions of 6M urea does not induce any observable modifications.

Following ultrafiltration, urea or another suitable agent is used. This is a separate chemical inactivation step to remove residual viral infectivity. After treatment, urea is removed by ultrafiltration on 10,000 dalton cut-off cassettes.

## Contaminants

The contaminants which are removed from biological fluids using the purification process of the invention include any materials whose sizes dictate that they be retained by the 100,000 dalton molecular weight filtration membrane during targeted filtration. Thus, the viral or other contaminants which are removed and disposed of in accordance with the invention are human and/or animal viruses of appropriate size.

Since gamma globulin and blue dextran are the retention markers for the filtering membranes employed, it can be said that the molecular weights of the contaminants retained on the membrane are slightly higher than 100,000 i.e., 110,000 or more.

Viral contaminants which can be removed using the process and filtration system of the invention include so-called "slow" viruses, such as scrapies virus, Creutzfeld Jakob Disease Virus (CJD) and the like.

Other contaminants which can be removed from biological fluids and extracts using the filtration system of the invention include common viruses, e.g., AIDS (HIV), SV 40, hepatitis, as well as virus-like particles, e.g., prions, and the like. Combinations of such impurities can also be removed.

After their removal from the purified hormone or other fluid, the contaminants retained on the filtration membrane are washed therefrom and disposed of by suitable means, e.g., incineration, burial, autoclaving or inactivation.

The purified fluid is then subjected to suitable techniques for storage, handling, packaging or other processing of the fluid. Such processing may include freeze drying (i.e., lyophilization), solvent precipitation, refrigeration, freezing (as is or in solution), urea treatment, and the like. One or more of these techniques and/or other processing techniques can be employed.

The following examples further illustrate the invention.

## Example 1

This example provides the introduction of the scrapie agent to Serono's hGH (commercially available as "GRORM") and compares the exclusion capability of both the 25 nM and 100,000 MW ultrafilters; and the effect of further treatment with 6M urea.

## PROCEDURE

## Virus Source

Hamster brains infected with the 263K strain of scrapie will be used as the "slow virus" contaminant. It has been shown that the scrapie agent has the same biological and physiochemical characteristics as the CJD agent. The hamster adapted strain of scrapie has been chosen because of its short incubation period (55-60 days after intracerebral (IC) inoculation of the highest dose of virus) and because of its great virus

infectivity titer $10^{10}$ $LD_{50}$/g of brain).

The hamster-adapted 263K strain of scrapie, isolated by Kimberlin and Walker(1977), was passed twice in golden Syrian hamster by intracerebral (IC) inoculation with 10% scrapie-infected brain homogenate in phosphate-buffered saline (PBS), pH 7.4. Hamster brains were aseptically removed from terminally ill animals and immediately frozen at -80 degrees centigrade. Brains were thawed and homogenized in sterile phosphate buffered saline at pH 7.4 to a final concentration of 10%. The suspension was centrifuged at 1500 g for 30 minutes and the supernatant divided into 5ml aliquots and stored frozen at -80 degrees centigrade.

Scrapie-Contaminated-hGH Purification

Lyophilized hGH (90% pure monomeric human growth hormone proteins) is dissolved in 10.8 ml of 0.01 M $NH_4HCO_3$, pH 8.0; 1.2 ml of the 10% clarified suspension of hamster brain infected with the 263 K strain of scrapie is sonicated (3x5 seconds) and added to the hGH solution to give a final volume of 12 ml. A 2 ml sample is withdrawn to determine the infectivity titer.

```
                       High Titer Challenge
             (1.2 ml) Clarified Scrapie Hamster Brains
           & (10.8 ml) Lyophilized Serono hGH (324 mg)
                                  |
                       (12 ml Total Volume
                                  |
           1st Assay Titration  Expected Titer 5X10 /0.05 ml)
                                  |
                                (-2 ml) Titer Undiluted thru 10
                                (132 hamsters)

        (5ml)                  (5ml)
          |----------------------|--------------------|
  100,000 MW Filtration                    25nM Filtration
  2nd Assay Titer (-2 ml)                  5th Assay Titer (-2 ml)
  Undil. to 10 (72 hamsters)               Undil. to 10 (72 hamsters)
          |                                         |
        (3 ml)                                    (3 ml)
          |                                         |
  6 M Urea                                 6 M Urea
  Overnight at 4 degrees C                 Overnight at 4 degrees C
  3rd Assay (Total Volume)                 6th Assay (Total Volume)
  Undil. Only (60 Hamsters)                Undil. Only (60 Hamsters
```

1st Assay:

Expected titer approximately $10^8$ $LD^{50}$/ml or 5x$10^6$ $LD_{50}$/0.05 ml. The sample is diluted 10 fold up to $10^{-10}$. 0.05 ml aliquots of dilutions from undiluted to $10^{-10}$ are inovulated (IC) in to 12 hamsters. (Number of Animals: 12 x 11 = 132).

This diluent added in this and subsequent assays was 0.01 Molar $NH_4HCO_3$ buffer, pH 8.0.

The remaining 10 ml are divided into two equal aliquots of 5 ml. The 5 ml aliquot (2nd Assay Titration) is diluted up to 100 ml with 0.01 M $NH_4HCO_3$ buffer passed through a Millipore 100,000 MW cut-off ultrafilter. The filtrate is concentrated by ultrafiltration and lyophilization, and the final volume is adjusted to 5 ml. A 2 ml sample is withdrawn for the 2nd Assay Titration.

2nd Assay

The sample is 10 fold serially diluted up to $10^{-5}$ 0.05 ml aliquots of the undiluted and diluted samples are inoculated (IC) in the 12 hamsters. (Number of Animals: 12 x 6 = 72).

The remaining 3 ml filtrate is treated with 6 Molar Urea as follows:

1) 1.40 grams of urea (Mol. wt. 60.02, specific volume 0.756) is added to the 3 ml aliquot to get a final concentration of 6 Molar. After overnight treatment at 4 degrees centigrade, the solution is dialyzed by ultrafiltration against $NH_4 HCO_3$ buffer to remove urea. The solution is then lyophilized and resuspended in 3 ml of 0.01 M $NH_4 HCO_3$, pH 8.0.

3rd Assay

The total volume, undiluted is injected (IC) into hamsters. (Number of Animals: 60).

2) The second 5 ml aliquot is passed through a series of filters consisting of 220 nM, 40 nM and 25 nM. The filtrate is concentrated by ultrafiltration and lyophilization, and the final volume is adjusted to 5 ml, with 10 mm ammonium bicarbonate. A 2 ml sample is withdrawn for the 5th assay titration.

5th Assay

The sample is 10 fold serially diluted up to $10^{-5}$ using Phosphate Buffered Soline (PBS) containing 100 units/ml Penicillin and 0.1 mg/ml Streptomycin. 0.05 ml aliquots of the undiluted and diluted samples are inoculated (IC) in 12 hamsters. (Number of Animals: 12 x 6 = 72).

The remaining 3 ml filtrate is treated as follows:

3) 1.40 grams of urea (Mol. wt. 60.02, specific volume 0.756) is added to the 3 ml aliquot to get a final concentration of 6 Molar. After overnight treatment at 4 degrees centigrade, the solution is dialyzed by ultrafiltration against $NH_4 HCO_3$ buffer to remove urea. The solution is then lyophilized and resuspended in 3 ml of 0.01 M $NH_4 HCO_3$, pH 8.0.

6th Assay

The total volume, undiluted, is injected (IC) in hamsters. (Number of Animals: 60).

The ultrafiltration operation at 100,000 MW was conducted using tangential filtration in a Minitan system (Millipore) equipped with 8 packets of membranes at about 0.5 bars pressure and 4 degrees C. The 25 nm filtration was conducted in a Millipore 600 ml filling unit equipped with a 0.47 mm Swinnex Filter Holder Millipore.

Results

The effects on residual laboratory contamination using the titration and filtration products described above are reported in the following tables.

Table 1

| 1st Assay Titration | | | | |
| --- | --- | --- | --- | --- |
| Dilution | Incubation time (days) | No. of Inoculated animals | Dead | Alive |
| none (Undiluted) | 63.0 ± 0 | 12 | 12 | 0 |
| $10^{-1}$ | 73.5 ± 0.5 | 12 | 12 | 0 |
| $10^{-2}$ | 84.0 ± 0 | 12 | 12 | 0 |
| $10^{-3}$ | 93.3 ± 5.4 | 12 | 12 | 0 |
| $10^{-4}$ | 111.5 ± 3.3 | 12 | 11 | 1 |
| $10^{-5}$ | 114.0 ± 4.0 | 11 | 8 | 3 |
| $10^{-6}$ | 125.7 ± 2.5 | 11 | 3 | 8 |
| $10^{-7}$ | 149 | 11 | 1 | 10 |
| $10^{-8}$ | No Symptoms | 11 | 0 | 11 |
| $10^{-9}$ | No Symptoms | 12 | 0 | 12 |
| $10^{-10}$ | No Symptoms | 11 | 0 | 11 |
| The infectivity titer calculated acording to Reed and Muench method gives an $LD_{50}$ of $10^{5.5}$/0.05 ml ($10^{8.8}$/gram of brain). | | | | |

Table 2 2nd Assay (100,000 MW Filtration)

| Dilution | Incubation time (days) | No. of Inoculated animals | Dead | Alive |
|---|---|---|---|---|
| none (Undiluted) | $90.7 \pm 0.8$ | 12 | 12 | 0 |
| $10^{-1}$ | $103.3 \pm 2.5$ | 12 | 12 | 0 |
| $10^{-2}$ | $113.2 \pm 8.1$ | 12 | 12 | 0 |
| $10^{-3}$ | $118.5 \pm 7.0$ | 12 | 5 | 7 |
| $10^{-4}$ | $139.0 \pm 18.4$ | 12 | 2 | 10 |
| $10^{-5}$ | 133 | 11 | 1 | 10 |
| The invectivity titer calculated according to Reed and Muench method gives an $LD_{50}$ of $10^{3.1}/0.05$ ml ($10^{6.4}$/gram of brain). After 100,000 MW Filtration there is a decrease in the infectivity titer of 2.4 logs. | | | | |

## Table 3: 3rd Assay (100,000 MW Filtration + 6 M Urea)

The total volume of 3rd Assay has been intracerebrally inoculated in 58 wealing hamsters. One animal died soon after the inoculation procedure. 46 hamsters showed clinical signs of scrapie diseases $126.0 + 13.9$ days after inoculation. The other 11 animals are alive and with no clinical signs of scrapie 180 days after the inoculation. The mortality rate is 80.7% and the infectivity titer calculated with the incubation period gives and $LD_{50}$ of $10^{2.9\pm1.3}$/gram of brain.

After 100,000 MW Filtration + 6M Urea there is a decrease in the infectivity titer of $5.9 \pm 1.3$ logs.

Table 4

| 5th Assay (25 nm Filtration) | | | | |
|---|---|---|---|---|
| Dilution | Incubation time (days) | No. of Inoculated animals | Dead | Alive |
| none (Undiluted) | $87.0 \pm 0$ | 12 | 12 | 0 |
| $10^{-1}$ | $99.1 \pm 2.0$ | 12 | 12 | 0 |
| $10^{-2}$ | $112.1 \pm 4.0$ | 12 | 12 | 0 |
| $10^{-3}$ | $133.1 \pm 10.0$ | 12 | 9 | 3 |
| $10^{-4}$ | 135 | 12 | 1 | 11 |
| $10^{-5}$ | 127 | 12 | 1 | 11 |
| The infectivity titer calculated according to Reed and Muench method gives an $LD_{50}$ of $10^{3.4}/0.05$ ml ($10^{6.7}$/gram of brain).<br>After 25 nm Filtration there is a decrease in the infectivity titer of 2.1 logs. | | | | |

## Table 5: 6th Assay (25 nm Filtration + 6 M Urea)

The total volume of the 6th Assay has been intracerebrally inoculated into 53 wealing hamsters. One animal died soon after inovulation procedure. 50 hamsters showed clinical signs of scrapie disease 111.7 + 10.6 days after inoculation. The other 2 animals are alive and with no clinical signs of scrapie 180 days after the inoculation. The mortality rate is 96.2% and the infectivity titer calculated with the incubation period gives an $LD_{50}$ of $10^{4.2 \pm 1}$/gram of brain.

After 25 nm Filtration + 6 M Urea there is a decrease in the infectivity titer of $4.6 \pm 1.0$ logs.

The samples which were processed in accordance with the invention (i.e., ultrafiltration using the 100,000 MW cut-off membrane with or without the additional urea contacting step) showed lower rates of infectivity. Generally, by means of the present ultrafiltration infectivity was reduced 99.6%, i.e., comparable to values obtained when the samples were filtered using a 25nm filtration system.

The effects of filtration on infectivity are presented in Table 6.

Table 6

| Experimental Procedure(s)-samples | Infectivity[a] titres (log) | Decrease of titres (log) |
|---|---|---|
| Control - A | 9.0 | --- |
| Filtration (100,000 MW) - B | 6.8 | 2.2 |
| Filtration (25 nm) - C | 7.0 | 2.0 |
| Filtration (B) + Urea - BU | 3.4 +/- 0.2 (b) | 5.6 +/- 0.2 |
| Filtration (C) + Urea - CU | 4.6 +/- 0.2 (b) | 4.4 +/- 1.3 |

(a) Infectivity titres are expressed as - $\log_{10}$ $LD_{50}$ i.c. units/g of brain (Am. J. Hyg. 1938; 27; 493-97).

(b) Infectivity titres measured by incubation time (J. Comp. Path. 1969; 79; 15-22).

Exposure of the 100,000 MW cut-off filtrate to 6 M urea (BU) had a stronger inactivation effect than when applied to the 25 nm filtrate (CU). The mean number of days from inoculaton until the appearance of clinical signs was 136.5 +/- 4.0 (mean + SEM) for BU and 114.2 +/- 2.3 days for CU. The difference was statistically significant by the students T-test and Wilcoxon test at the level $p < 0.0001$. Number of affected animals for BU is 54 out of 58 animals and for CU, 52 out of 52. This difference is by chi square analysis significant, P = 0.0207. The reason why the combination of filtration by a 25 nm filter + urea showed a lower level of Scrapie elimination than 100,000 cut-off filtration + 6 M urea, cannot be explained at this stage.

In the present experiment, the 2 combined procedures (100,000 MW cut-off filtration plus purification of filtrate with 6 M urea) were applied to a highly infective brain homogenate with an infectivity titer of 10 (8.8) logs/gm. These 2 procedures reduced the infectivity by approximately 6 logs without the application of any preceding or subsequent purification steps.

When the steps of the invention are applied to already purified hGH they further increase the margin of safety of hGH or any other product on which they might be employed. Therefore, even if modern technologies are employed, the product should still be submitted to these 2 procedures since they significantly increase the safety of the product. Accordingly, co-joint use of the filtration operation or the filtration operation with the addition of urea alone and with other purification techniques is contemplated.

Example 2

This example shows the treatment of a solution of human menopausal gonadotropin (HMG) derived from urine using the 100,000 dalton cut-off membrane.

120 ml of urinary HMG solution (in water) were taken from the concentrated solution before the final precipitation step of HMG production. It has the following characteristics:

Quantity = 120 ml. coming from = 3281 liters of urine

Analysis of solution:

Proteins = 4.17 mg/ml Total proteins = 500 mgr.

FSH (Biol.) = 1258 UI/ml corresponding to = 46 UA/lt. urine

pH = 7.3

This solution was diluted to 1 lt by $NH_4 HCO_3$, 01 M, pH8.

The final solution was tangentially filtered using a 100,000 dalton cut-off Millipore PTHK membrane in a cassette at 1 kg/cm$^2$ pressure and 6 ± 2 degrees celsius temperature. The filtration has been performed until a volume of 200 ml was obtained; after that the concentration was repeated twelve times adding each time 200 ml of $NH_4 HCO_3$, 0.1 M, pH = 8 - see flow chart.

Starting solution = 500 mg. protein

After cut-off <u>100,000</u>

$$\text{Filtrate} = \text{D.O.} \times \text{ml.} = 0.125 \times 3450 = 431 \text{ mg.}$$
$$\text{Retained part} = \text{D.O.} \times \text{ml.} = 0.4 \times 200 = 80 \text{ mg.}$$
--------
$$\text{Total} = 511 \text{ mg.}$$

volume $\Big\langle$ filtrate = 3450 ml
retained = 200 ml

D.O. $\Big\langle$ filtrate = 0.125
retained = 0.4

$$\text{Efficiency} \frac{431}{511} \times 100\% = 84.3$$

The filtrate obtained above was passed over a 10,000 dalton cut-off membrane and yielded:
Total proteins involved = 431 mg.
Recovery of proteins:
-Collected concentrated = O.D.x ml = 0.83 x 410 ml = 340 mg.
-Filtrate = 0
Proteins loss from cut-off of 10,000 = 431 - 340 = 91 mg.

$$\text{Efficiency} = \frac{340}{431} \times 100\% = 78.9\%$$

The flow-sheet of Example 2 follows:

```
                        FLOW - SHEET
            ULTRAFILTRATION CUT-OFF 100,000 MW
```

FLOW - SHEET
ULTRAFILTRATION CUT-OFF 100,000 MW

NH₄HCO₃ 0,1 M — 880 ml.

Starting Solution 120 ml.

Dilution to 1 lt. pH=8

CONCENTRATION BY ULTRAFILTRATION CUT-OFF 100,000

200 ml. x 12 times    conc. 200 ml.

FILTRATE → 800 ml. O.D. = 0.217   35   Progressive Recovery %

12 times    ULTRAFILTRATION CUT-OFF 100,000

FILTRATE → 200 ml. O.D. = 0.198   43

EtOH    Retained 200 ml. O.D. = 0.4

200 ml. O.D. = 0.145   49

200 ml. O.D. = 0.123   54

PRECIPITATION COLLECTING

200 ml. O.D. = 0.099   58

A (68.6 mg) corresponding to : 0.02 mg/lt.Urine

200 ml. O.D. = 0.089   61.3

200 ml. O.D. = 0.083

200 ml. O.D. = 0.078

Note: (1) SDS Electrophoresis show less low molecular weights

(2) O.D. = optical density

200 ml. O.D. = 0.058

200 ml. O.D. = 0.056

200 ml. O.D. = 0.039

200 ml. O.D. = 0.04

200 ml. O.D. = 0.031

TOTAL FILTRATE

(from cut-off 100,000)

3450 ml.

CONCENTRATION
cut-off 10,000

FILTRATE

PRECIPITATION
COLLECTING

B

341 mgr.

Weight yield = 0,104 mgr/lt.U.

FSH activity = 262 IU/lt. (BIOASSAY)

Example 3

The following table demonstrates the yields obtained using the process of the invention to filter a part of one factory batch in comparison with the other part normally worked.

The FSH content at starting step was: 46 I.U./lt urine.

## Summary of experiment compared to a typical factory batch of Follicle Stimulating Hormone (FSH)

|  | Factory Batch | A Retained | B Filtrate |
|---|---|---|---|
| Yield mg./lt. Urine | 0.166 | 0.02 | 0.104 |
| Yield FSH UI/lt. Urine | 36.7 | -- | 27.2 |
| FSH specific activity UI/mgr. | 235.7 | -- | 262 |
| % Yield with reference to the starting solution (46 UI/lt. Urine) | 79.5 | -- | 59 |

Total yield of the ultrafiltration process cut-off 100,000 plus cut-off 10,000 with reference to the factory yield:

$$\frac{27.2}{36.7} \times 100 = 74\%$$

### Example 4

Using procedures similar to those employed in Example 1, SV40 virus can be removed from human urine. The results of animal tests are expected to show decreased infectivity when 100,000 dalton cut-off membranes are used. Dilution and/or the addition of urea should assist in lowering infectivity.

### Example 5

Using procedures similar to those employed in Example 1, AIDS virus can be removed from urine and other human biological fluid. The results of animal tests should show that filtration with the membrane of the invention decreases infectivity. Furthermore, dilution and/or the use of urea generally should help lower the activity of any residual virus in the purified material.

### Example 6

The following text is taken from a protocol developed for studying the removal of Simian Virus 40 from human growth hormone by a PTHK membrane purification process.

### PURPOSE

To determine the virus properties of the PTHK membrane using Simian Virus 40 (SV40) appearing in the permeate after filtration.

The initial study will evaluate the influence of critical performance variables on the removal of a model conventional virus, SV-40, and simultaneous passage of human growth hormone using Millipore PTHK membrane fabricated into a Minitan device. The first objective is to evaluate the effect of the batch volume to membrane surface area ratio when operated at 0.5 Bars transmembrane pressure. This operating condition matches production operation at exactly $1/10^{th}$ scale. The second objective is to evaluate the effect of operating conditions, the ratio of permeate flowrate to retentate flowrate, relaxing the volume to surface area ratio constraint. Two operating conditions will be evaluated in this manner. All other processing operations, i.e. urea treatment, number of wash steps, etc., will be designed to simulate current manufacturing practice.

TEST ARTICLE

Source : Ares-Serono
Name : PTHK in Minitan configuration
Source : Millipore

POSITIVE CONTROL

Simian Virus 40 (SV40) Source: Virology Department, Microbiological Associates, Inc.

TEST SYSTEM

Test System: Vero cells for SV40 virus assay maintained in the Biotechnology Services Department Laboratory. These procedures for monitoring the change in the amount of SV40 present in samples utilize standard virus quantitation procedures.

EXPERIMENTAL DESIGN AND PROCEDURES

A. The test article solution will be spiked with SV40 and carried through a filtration scheme which will include filtration at different operating conditions. Samples will be collected at several steps during the filtration procedure, as specified below, and the samples will be stored at -70 degrees centigrade until assayed for plague titer.
B. The set-up and performance of the filtration procedures will be the responsibility of the sponsor. The testing facility will provide the SV40 with which to spike the sample and will take the samples from the specified purification steps for subsequent plaque assay. The target concentration of the spiking virus will be approximately $10^6$ pfu/ml after dilution in the test material.
The plague assays will be performed at appropriate dilutions to yield accurate titers, (maximum of 3 per sample) in balanced salt solution as specified below.

PROCEDURES

A. Protocol A

1.0 Lyophilized h-GH will be reconstituted in 285 mls of 0.01M ammonium bicarbonate buffer to 2 mg/ml. Three 1 ml samples will be retained for SV-40 and h-GH assay and stored at -70 degrees centigrade (A1).
2.0 The 285 mls of stock h-GH solution will be inoculated with 15 mls of stock SV40 solution at approximately $2 \times 10^7$ pfu/ml (a 1/20 dilution) to a final titer of $10^6$ pfu/ml. Three 1 ml samples of this inoculated solution will be retained and for SV-40 and h-GH assays and stored at -70 degrees centigrade (A2). This inoculated feed will be separated into 3 equal aliquots of 100 mls each for introduction into the filtration apparatus of protocols A1, A2 and A3.

A.1. Protocol A1

1.0 100 mls of inoculated feed will be filtered through a PTHK minitan (8 stack) at 0.5 Bars transmembrane pressure. A series of 9 washings will be performed to recover the totality of the h-GH. Each washing step will consist of filtering 100 mls of solution until the starting volume is reduced to between 15-20 mls. This remaining volume is rediluted up to 100 mls with .01M ammonium bicarbonate solution prior to the next washing step. After the initial filtration and 9 washes, a final volume of 800-850 mls of filtered product will have been generated.

2.0 Three 1 ml samples of this filtered product will be retained and stored at -70 degrees centigrade for analysis (A3) of both SV-40 and h-GH.

3.0 100 mls of this filtered product will be concentrated to approximately 10 mls using a PTGC Minitan S system. This 10 mls will be retained (A4), stored at -70 degrees centigrade, and assayed for SV-40 and h-GH.

4.0 Urea will be added to the remaining 700-750 mls of PTHK filtered h-GH to achieve a 6M urea concentration. This solution will be stored overnight (16 hrs) at 4 degrees centigrade.

5.0 A series of 7 0.01m ammonium bicarbonate washes using a PTGC Minitan will be performed to remove the urea after treatment. The initial 750 ml volume will be reduced to 100 mls. One liter of 0.01M ammonium bicarbonate is then added. This solution volume is again reduced to 100 mls. This wash procedure is repeated a total of 7 times to remove the urea. After the washes the system is washed with up to 300 mls of 0.01M ammonium bicarbonate to recover all of the h-GH.

6.0 Three 4 ml samples of this final processed h-GH product will be retained and stored at -70 degrees centigrade for later SV40 and h-GH analysis (A5).

### A.2. Protocol A2

1.0 100 mls of inoculated feed will be filtered through a PTHK Minitan (1 stack) at a 1:1 filtrate to permeate flowrate split. The h-GH solution will be washed as in Protocol A.1.1 at these conditions.

2.0 Three 1 ml samples of filtered product will be retained (A6) and stored at -70 degrees centigrade for SV-40 and h-GH analysis.

### A.3. Protocol A3

1.0 100 mls of inoculated feed will be filtered through a PTHK Minitan (1 stack) at a 5:1 filtrate to permeate flowrate split. The h-GH solution will be washed as in Protocol A.1.1 at these conditions.

2.0 Three 1 ml samples of filtered product will be retained (A7) and stored at -70 degrees centigrade for SV-40 and h-GH analysis.

### C. Controls

### C.1. Freeze - Thaw Control Study

Obejctive: To determine if SV40 at concentrations approximately that of the samples in Protocol A can be frozen at -70 degrees centigrade and thawed recovering virus titer.

1.0 Stock SV40 solution at 107 pfu/ml will be serially diluted to, 10, $10^2$, and $10^3$ pfu/ml in .01M ammonium bicarbonate.

2.0 1 ml samples of each concentration will be allowed to stand at room temperature for a period of time equal to that of Protocol A (about 4 hours) and immediately assayed for SV-40 without further handling (C1).

3.0 Aliquots of all three concentrations will be frozen at -70 degrees centigrade for a period of time equal to the storage time used in Protocol A1.

4.0 All three concentrations will be thawed to room temperature and assayed for SV-40 (C2).

### C.2. Buffered Toxicity (ammonium bicarbonate and h-GH)

Objective: To determine the toxicity of both 0.01M ammonium bicarbonate buffer and h-GH in this buffer to the SV40 plaque assay.

1.0 A solution of 0.01M ammonium bicarbonate will be spiked with stock SV-40 to a concentration of $10^4$ pfu/ml. This sample will be assayed for SV-40 (C8) and the titer compared with that of the SV-40 standard curve.

2.0 The 0.01M ammonium bicarbonate will be serially diluted 10x (sample C9) and 100x (sample C10) in tissue culture medium. Each of these samples will be inoculated with stock SV-40 to a concentration of $10^4$ pfu/ml and assayed.

3.0 Stock SV40 at $10^7$ pfu/ml will be diluted 1/20 into stock h-GH in 0.01M ammonium bicarbonate to make the final SV40 concentration of about $10^6$ pfu/ml. This solution will be assayed (sample C7) for SV-40 titer.

## C.3. 6M Urea Control

Objective: To determine the virus inactivation capability of 6M Urea treatment of SV40 virus.

1.0 5 mls of stock SV40 solution will be used to inoculate 95. mls of stock h-GH solution at 2 mg/ml in 0.01M ammonium bicarbonate to a final concentration of $10^6$ pfu/ml SV40.

2.0 Three 1 ml samples will be drawn (C3) and stored at -70 degrees centigrade for SV-40 and h-GH analysis.

3.0 Urea will be added to a final concentration of 6M urea and allowed to stand for 16 hours at 4 degrees centigrade.

4.0 The urea will be removed by filtration using a PTGC Minitan as per Protocol A.1.5.

5.0 Three 4 ml samples of this filtered solution will be retained for SV-40 and h-GH analysis and stored at -70 degrees centigrade (C4).

## C.4. 10K Dalton UF Control

Objective: To validate the PTGC Minitan.

Note: Samples from this experiment will be stored and analyzed only if required, i.e., Sample A4 demonstrates significant SV40 loss.

1.0 5 mls of stock SV40 solution will be mixed with 95 mls of stock h-GH in 0.01M ammonium bicarbonate at 2 mg/ml to a final concentration of about $10^4$ pfu/ml SV40.

2.0 Three 1 ml samples will be retained (C5) and stored at -70 degrees centigrade for SV-40 analysis.

3.0 The inoculated solution will be concentrated using the PTGC Minitan to a final volume of about 10 mls.

4.0 Three 1 ml samples of the 10 ml volume will be retained and stored at -70 degrees centigrade (C6).

## Materials

1.0 Stock SV40 at $2 \times 10^7$ pfu/ml.

2.0 Lyophilized h-GH:1.5 grams.

3.0 Ammonium bicarbonate.

4.0 Urea.

5.0 Sterile water.

6.0 Filter units.

Protocol A:

10 PTHK Minitan Packets

4 PTGC Minitan Packets

4 Minitan units

Membrane :

Two membranes will be selected from PLMK, PKMK and PZHK.

Selection will be made on the basis of h-GH passage.

h-GH passage will be predetermined at Millipore in Bedford using h-GH sent to Millipore from Ares-Serono.

## Protein Determination

The protein determination on the feed and permeate solutions will be performed by the dye-binding method of Brandford (Anal. Biochem. 72,248 (1976)).

BioRad protein assay dye reagent (500-006) will be used. The standard will be purified pituitary-hGH (1 mg/ml) stored at -20 degrees centigrade or below.

1. The assay procedure is as follows:

a. Standard Curve

The standard curve will be determined for each new bottle of dye reagent concentrate.

|  | hGH ($\mu$g/ml) | | | | |
|---|---|---|---|---|---|
|  | 0 | 5 | 10 | 15 | 20 |
| Pituitary-hGH (0.1 mg/ml) | 0 | 50 | 100 | 150 | 200 |
| Distilled water | 800 | 750 | 700 | 650 | 600 |
| Dye reagent concentrate | 200 | 200 | 200 | 200 | 200 |

* The columns represent volumes in $\mu$l.
* Assays are made in triplicate, except the blank solution.
* After an incubation of 15 minutes, the OD (595 nm) of standard samples versus reagent blank are recorded.
* The standard curve is obtained by plotting the OD (595 nm) versus the concentrations of standard.
* The slope of the standard curve is then calculated.

b) hGH Sample Preparation

* The hGH solution is diluted with distilled water to get about 1-2 mg protein/ml.
* An appropriate volume of diluted sample containing 5 to 20 $\mu$g is placed in the test tubes. The total volume is made up to 800 with water.
* 200 $\mu$l of dye reagent concentrare are then added and mixed.
* The OD (595 nm) is recorded after 15 min incubation against reagent blank.

2. Determination of hGH concentration:

$$\frac{OD \ (hGH) \ x \ slope \ of \ standard \ curve}{volume \ of \ diluted \ sample} = \ ... \ \mu g/ml$$

Sample Identification

| Sample Code | Sample Identification | Volume per Sample (mls) | Estimated conc'n (pfu/ml) | Number of Dilutions |
|---|---|---|---|---|
| A1 | Stock reconsituted h-GH | 1 | 0 | 1 |
| A2 | Inoculated h-GH feed h-GH | 1 | $10^6$ | 3 |
| A3 | h-GH post PTHK filtration via protocol Al | 1 | $10^3$ | 3 |
| A4 | h-GH post PTHK filtration concentrated 10x via 10,000 dalton ultra-filtration | 1 | $10^4$ | 3 |
| A5 | Urea washed h-GH solution after urea removal | 4 | 0 | 1 |
| A6 | h-GH post PTHK filtrtation via Protocol A2 | 1 | $10^4$ | 3 |
| A7 | h-GH post PTHK filtratin via Protocol A3 | 1 | $10^4$ | 3 |

EP 0 307 373 B1

## Sample Identification

| Sample Code | Sample Identification | Volume per Sample (mls) | Estimated conc'n (pfu/ml) | Number of Dilutions |
|---|---|---|---|---|
| C1 | Freeze/Thaw control | | | |
| C1a | Stock SV40 diluted to $10^3$ pfu/ml | 1 | $10^3$ | 3 |
| C1b | Stock SV40 diluted to $10^2$ pfu/ml | 1 | $10^2$ | 3 |
| C1c | Stock SV40 diluted to 10 pfu/ml | 1 | 10 | 3 |
| C2 | Thawed controls | | | |
| C2a | $10^3$ pfu/ml | 1 | $10^3$ | 3 |
| C2b | $10^2$ pfu/ml | 1 | $10^2$ | 3 |
| C2c | 10 pfu/ml | 1 | 10 | 3 |
| C3 | 6M Urea Control Inoculated hGH feed at $10^6$ pfu/ml | 1 | $10^6$ | 3 |
| C4 | Urea treated h-GH after urea removal via 10,000 dalton UF wash | 4 | 0 | 1 |
| C5 | 10,000K UK Control: Inoculated h-GH feed at $10^4$ pfu/ml | 1 | $10^4$ | Retain |
| C6 | Retentate after 10,000 dalton UF 10 fold concentration | 1 | $10^5$ | Retain |
| C7 | Buffer toxicity: Inoculated h-GH at $10^6$ pfu/ml | 1 | $10^6$ | 3 |
| C8 | Buffer toxicity: Ammonium bicarbonate buffer | 1 | $10^4$ | 3 |
| C9 | Ammonium bicarbonate diluted 10x in Tissue Culture medium | 1 | $10^4$ | 3 |
| C10 | Ammonium bicarbonate diluted 100x in Tissue Culture medium | 1 | $10^4$ | 4 |

Via the examples above, it is shown that filtration using the 100,000 dalton cut-off filter membrane in a cassette system, with purified extract yields purified hormonal extracts of human or other mammalian origin from which viral contaminants are effectively removed. Thus, applicants' is an industrially applicable process by which biological fluids can be purified on a commercial scale. Furthermore, the use of urea as a decontaminant and the other techniques, e.g., dilution techniques, disclosed herein substantially reduce the likelihood of viral infections being spread to recipients using biological fluids which have been treated in accordance with the invention.

Reasonable variation, such as those which would occur to a skilled artisan, can be made herein without departing from the scope of the invention.

**Claims**

1. A process for substantially reducing the viral infectivity of or removing viral contaminants from a biological product without significant loss thereof characterised in that it comprises the tangential flow filtration of a fluid containing the biological product through an ultrafiltration membrane having a 100,000 dalton cut-off and the recovery of the biological product from the filtrate.

2. A process as claimed in claim 1 wherein the membrane has the following specifications:

| Retention: | Blue Dextran | ≧ 99% |
| | IgG | ≧ 95% |
| Passage: | Albumin | ≧ 60% |

3. A process as claimed in claim 1 or claim 2 wherein the filtered biological product is contacted with urea.

4. A process as claimed in any of claims 1 to 3 wherein the biological product is human growth hormone.

5. A process as claimed in any of claims 1 to 4 wherein the ultrafiltration membrane reduces viral infectivity by at least 99.6% while allowing product recovery of at least 90%.

**Patentansprüche**

1. Verfahren, um die virale Infektivität eines biologischen Produktes ohne signifikanten Verlust des Produkts wesentlich zu verringern oder virale Kontaminanten aus einem biologischen Produkt ohne signifikanten Verlust des Produkts im wesentlichen zu entfernen,
**dadurch gekennzeichnet, daß**
es die Tangentialstromfiltration einer das biologische Produkt enthaltenden Flüssigkeit durch eine Ultrafiltrationsmembran mit einem 100.000 Dalton Rückhaltevermögen und die Wiedergewinnung des biologischen Produktes aus dem Filtrat umfaßt.

2. Verfahren nach Anspruch 1, wobei die Membran die folgenden Merkmale aufweist:

| Retention: | Dextranblau | ≧ 99% |
| | IgG | ≧ 95% |
| Durchlauf: | Albumin | ≧ 60% |

3. Verfahren nach Anspruch 1 oder 2, wobei das gefilterte biologische Produkt mit Harnstoff in Kontakt gebracht wird.

4. Verfahren nach einem der Ansprüche 1 bis 3, wobei das biologische Produkt menschliches Wachstumshormon ist.

5. Verfahren nach einem der Ansprüche 1 bis 4, wobei die Ultrafiltrationsmembran die virale Infektivität um wenigstens 99,6% verringert, während eine Wiedergewinnung des Produkts von wenigstens 90% ermöglicht wird.

21

**Revendications**

1. Procédé pour réduire sensiblement le pouvoir infectant viral de/ou d'éliminer les contaminants viraux d'un produit biologique sans perte significative de celui-ci, caractérisé en ce qu'il comprend la filtration à écoulement tangentiel d'un fluide contenant le produit biologique à travers une membrane d'ultrafiltration ayant une coupure à 100 000 daltons et la récupération du produit biologique à partir du filtrat.

2. Procédé selon la revendication 1, dans lequel la membrane a les spécifications suivantes:

| Rétention : | Bleu de dextran | ≧ 99% |
|---|---|---|
| | IgG | ≧ 95% |
| Passage : | Albumine | ≧ 60% |

3. Procédé selon la revendication 1 ou 2, dans lequel le produit biologique filtré est en contact avec de l'urée.

4. Procédé selon l'une quelconque des revendications 1 à 3 dans lequel le produit biologique est l'hormone de croissance humaine.

5. Procédé selon l'une quelconque des revendications 1 à 4, dans lequel la membrane d'ultra-filtration réduit le pouvoir infectant viral d'au moins 99,6% tout en permettant la récupération du produit à raison d'au moins 90%.